# EUROPEAN PATENT APPLICATION

(11) **EP 4 332 212 A1**
(43) Date of publication of application: **06.03.2024**
(21) Application number: 22193389.8
(22) Date of filing: 01.09.2022
(51) Int. Cl.: C12M 3/00, C12M 1/00, B01D 11/04, C12M 1/08

(54) **MULTIPHASE BIOCATALYTIC REACTOR AND SEPARATOR SYSTEM**

(71) Applicant: Delft Advanced Biofuels B.V., 2613 AX Delft (NL)
(72) Inventor: OUDSHOORN, Arjan, 2613 AX Delft (NL); SCHAEFER, Dominik Andreas, 2613 AX Delft (NL); VAN LOTRINGEN, Marion, 2613 AX Delft (NL)
(74) Representative: V.O.

(57) **Abstract**

The present invention provides an integrated multiphase biocatalytic reactor and separator system (1), comprising, an upflow reaction section (2), a separation section (3), and a recycle flow section (4), wherein:
the upflow reaction section (2) comprises a gas phase inlet (12), arranged to introduce a gas phase (G) for lifting a reactor flow (C) comprising a first and a second phase liquid;
the separation section includes a first separation zone (31) configured to degas an effluent flow from the upflow section, and a second separation zone (32), i.e. a liquid-liquid phase separator, configured to separate the degassed effluent in a lower liquid phase (LP) and an upper liquid phase (UP), said second separation zone further comprising an outlet (15) for collecting the upper liquid phase and an outlet for recirculating the lower liquid phase back to the upflow reaction section via the recycle flow section (4).

## Description

### TECHNICAL FIELD AND BACKGROUND

The present disclosure relates to a multiphase biocatalytic reactor, in particular to an integrated multiphase biocatalytic reactor and separator system. The present disclosure further relates to a method of operating the biocatalytic reactor and separator system for converting a substrate to a product. The device and/or method can be of particular benefit to biocatalytic process or strain development, e.g. for larger scale multiphase biocatalytic reactor and separator systems and/or for strain selection or production.

There is a strong world-wide effort to provide more sustainable production routes, e.g. transition from fossil based substances to bio-based substances, for production of various organic substances, e.g. alcohols, esters, amino acids, carbohydrates, ethers, lipids, ketones, aldehydes, organic acids, pyridines. These substances can be used in a variety of applications for example as flavours, fragrances, cosmetic or food ingredients, nutraceutical products, bioinsecticides, specialty or commodity chemicals, or as biofuels, all as either sustainable replacement or as novel more sustainable product.

These more sustainable production routes include amongst others biocatalytic processes. It is known in the art to produce organic substances by biocatalytic processes such as fermentation and cell free conversions. In such a fermentation process, micro-organisms are used to convert a suitable substrate into an organic substance of interest. In a cell free conversion, enzymes convert a substrate into an organic substance of interest. As is generally known in the art, various micro-organisms and enzymes are known that can be used on an industrial scale for the production of a wide variety of organic substances. Generally known fermentative processes include the use of natural micro-organisms and/or genetically modified organisms, e.g. yeasts or bacteria, for the production of various organic substances, e.g. alcohols, esters, amino acids, carbohydrates, lipids, ketones, aldehydes, organic acids, ethers, pyridines, imines.

Organisms may be genetically modified to increase the product titre of a naturally produced organic substance and/or to enable a micro-organism to produce an organic substance that it does not produce naturally. E.g. a micro-organism may be modified by incorporating genes from a plant responsible for the production of an organic substance, e.g. a terpene, a terpenoid, not naturally produced by the micro-organism.

More specifically in the world wide effort to produce substances more sustainably there is a need to make more lipophilic products via biocatalysis. Most lipophilic products typically have limited solubility in water due to their 'oily' nature and some are toxic/inhibiting to micro-organisms. These lipophilic products and/or by-products can be inhibiting or toxic to the micro-organism, the products can be unstable, can be unstable under the reaction conditions in the overall reaction vessel or can stick to the micro-organism or any other surface. These effects create challenges for industrial production of these products and hamper the transition towards more sustainable industrial bio-based production. The production per process volume will be low resulting in high production cost per unit of product, creating competition disadvantage for bio-based production routes. Additionally different feed stocks may be used in the search for a cheaper carbon source. Second and third generation biomass may contain contaminants, which can be inhibiting and/or toxic to the micro-organism as well. High product, by-product and/or contaminant concentrations can be rate limiting to the conversion. They pose a limit on how efficient a biocatalytic process can be related to the overall production rate possible and also on product concentration being reached. Lower product concentrations lead to large reactor volumes and the diluted product containing phase increased effort needed to perform product recovery and/or purification to end product.

It is known in the art that biocatalytic and or fermentation production systems experiencing diluted product streams, inhibition, equilibrium limited, and/or product stability constrained systems benefit from in situ or integrated product removal. Product removal can occur through a continuous phase in and out flow. Often this has the form of a gas stripping or liquid phase extraction. At lab scale it can also involve membranes to separate the phases in a system.

WO2021/010822 describes a way to produce these substances in an economically feasible manner by using an integrated multiphase reactor system. The integrated multiphase bioreactor comprises a bioreactor compartment, a riser, and downcomer configuration above the bioreactor for degassing a multi-phase process flow. In the riser the net flow is upflow as it also is in the bioreactor and in the downcomer the net flow is downward. Downstream of the down-comer a liquid-liquid separator compartment is provided. In this reactor system the inhibiting and/or toxic and/or unstable substance(s) are removed from the reaction mixture by (continuous) substance removal through extraction and separation. Advantages include decoupling of the liquid phases in the reactor, targeting high production rates, good contact for liquid-liquid interaction, in situ product removal and separation and providing a concentrated product stream. An additional degree of freedom is introduced for the system by the addition of the substance removal functionality; it allows the system to operate at any desired operational work point, which can now be controlled. E.g. the concentration of the substance in the reaction mixture can be maintained by matching substance formation rate and substance removal rate.

The ability to control the concentration of the substance in the system at any given time point has several advantages. For example, it enables the measurement of substance inhibition properties under representative process conditions. Further, it can be used to compare the performance of micro-organisms (strains) under conditions of controlled substance concentration. Further, it can be used to compare fermentation process conditions under conditions of controlled substance concentration to determine which process conditions result in improved process performance (process optimization). Further, it makes it possible to compare the performance of individual or blends of organic liquids in continuous extractive fermentations, including but not limited to a) the interaction of 1) micro-organism, 2) organic liquid(s) and 3) substance, b) the separation of organic solvent(s) and fermentation broth and c) the extraction efficiency of individual or blends of organic solvents. Many more applications will be apparent to practitioners skilled in this art.

To widen and enhance optimal application of the integrated multiphase reactor system, fermentation process development, strain development and strain selection are required. Strain and fermentation development most preferably takes place with the target end reaction (industrial scale and conditions) in mind.

Reactor systems and reaction mixtures obtained in biocatalytic processes tend to be rather complex, especially when living micro-organisms, cell-mass of non-living micro-organisms, or sensitive enzymes are used. The reaction mixtures comprise typically the biocatalyst, substrate, nutrient (in case living organisms are used), the produced organic substance, and possibly side-products, e.g. a fermentation gas which is optionally produced (e.g. CO₂) or other substances secreted by the biocatalyst (in case living organisms are used). The complexity of the reaction mixture, chemical processes, microbiological processes and physical processes taking place in the reactor system can make it difficult to simulate the actual industrial conditions.

The system disclosed by WO2021/010822 is adding and removing an additional liquid phase, the product recovery liquid phase, allowing for in-situ product removal, which further challenges simulating or approximating at lab scale actual industrial conditions for strain and fermentation process development, because traditional lab scale systems either don't foresee product removal capability or provide alternative technologies with limited scalability or only have functionality for addition of the product recovery phase and therefore lack the additional degree of operational testing freedom this invention enables.

For strain development, e.g. strain modifications, and strain selection, testing conditions need to be applied to simulate industrial process conditions as good as possible, especially in regards to overall strain genotype and phenotype developing in ongoing continuous systems. Different conditions, introducing limitations and different strain selection pressures, hinder the process development and identification and selection of advantageous strains.

Traditionally, for fermentation process development chemostat systems are also applied for selection pressure on microbes. These operate under the premise that the effective growth rate is precisely at the liquid dilution rate of the system, thereby setting a specific growth rate as selection pressure. However chemostat systems are limited in control of product concentration, because growth and product concentration are typically not decoupled and therefore have limited functionality to manage inhibition.

At laboratory and large scale some systems with in situ product removal are known. For volatile products, these systems generally rely on gas stripping because gas and liquid phases can usually be easily separated in one vessel. However, the phase capacity of gas phases is (on mass basis) significantly lower than a liquid or solid phase, requiring processing of comparatively large volumes of gas. Further, gas stripping is in practice not suitable for high product formation operation (relative to the inhibition of the substance), because of maximum gas rate limitations that can be applied on the system industrially.

At lab scale, membrane aided in-situ reactor systems are used for enhanced process research and development. The membrane's predominant function here is biomass retention and/or keeping auxiliary liquid separated from the aqueous fermentation broth. However, applying steady state experimental conditions is difficult as concentrations in batch-like operations as to substrate, biomass and product concentrations vary throughout the fermentation, usually at fixed organic phase content. However, for development of fermentation processes at scale one would like to keep process conditions, e.g. the product concentration and other conditions, more constant.

Membrane systems configured for a separation process in which a solution is permeated through a membrane and subsequently extracted with a solvent making use of an osmotic effect (perstraction, whereby the biocatalytic liquid phase (aqueous) passes on one side of the membrane and the other side contacts a solvent for pulling on the product the membrane via osmotic effects) are less constrained in terms of continuous operation than conventional batch system, but introduce mass transfer constraints that do not translate at scale and/or further disadvantages such as membrane fouling.

Conventional (fed-)batch setup fermentations can be restricted by: addressing cell toxic product inhibition; long term operation testing for continuous or semi-continuous process development. Their batch nature also precludes them to operate at steady state conditions. This results in fermentation process development not optimal for continuous and continuous overlay fermentation systems.

Conventional (fed-)batch setup fermentations can also be applied in a liquid extractant form of operation and on top of the (fed-)batch restrictions they can further be limited by: amount of secondary liquid phase (organic phase) that can be added to the system and more complex concentration of the different components present in the system per phase impacting the chemical species present in the system and/or emulsion formation.

More and more, research is focussed on enhancing fermentation performance also focussing on continuous overlay and mode of operation of an integrated multiphase bioreactor and separator system, to obtain more economically viable fermentation systems, as traditional system are bottlenecked by product formation rate and titre limitations.

WO2017/149099 relates to a multiphase loop reactor. Herein two phases are fed and dispersed in a continuous phase, which can be the reaction phase. In an embodiment, one of the phases can be a gas phase the other a liquid phase. One dispersed phase moves co-current with the continuous phase, the other counter-current. Importantly, the dispersed phases are conveyed in spatially separated sections of the reactor. The respective dispersed phases do not interact with each other. The product is separated from the reaction mixture making use of phase dispersion and retention which imparts maintaining constant process conditions during use, e.g. for applying strain selection pressure.

One of the phases requires a lower rate of ascent than the other, resulting in different residence times of the continuous phase in each section and substantially differences interaction times between the continuous phase and each of the dispersed phases and thus fundamentally differs from the present disclosure.

### SUMMARY

The inventors realized that there remains a need for alternative methodologies and bioreactor systems for recovery of produced organic substance of interest from the reaction mixture. In particular there is a need for such a biocatalytic reactor and separation system that can be used for bio-based fermentation and process development at lab scale, especially to extractive liquid multiphase fermentation systems.

Strain and process development require methods and instruments to operate these extractive fermentation processes on lab scale because large scale testing has low throughput (number of tests that can be done in a certain timeframe) and involves high cost. State-of-the-art laboratory scale bioreactors allow the use of batch overlay extractive fermentation processes but continuous solvent removal has not been possible to control the product concentration and other fermentation parameters at any given time. This severely limits the development of economically attractive production strains and corresponding production processes because strains and processes cannot be operated under the conditions that are representative for production scale. For example, the performance of a production strain with increased productivity can be fully masked by product inhibition, severely limiting effective strain testing. Similarly, (by-)product accumulation during a fermentation may prematurely end product formation because (by-)product is not being removed, severely limiting opportunities for process optimization. It would therefore be highly desirable to be able to apply continuous solvent removal on lab scale to support strain and process development.

The present disclosure advantageously provides a system, in particular a system suitable for lab scale processing, that overcomes one or more of the disadvantages of known systems. Advantageously the biocatalytic reactor and separator system according to the present disclosure allows for process development, in particular at lab scale, which enables to apply continuous organic phase addition and removal from a biocatalytic reactor. Thereby a needed tool for biocatalytic (e.g. microbial and/or enzymatic) process development activities for industrial application of bio-based processes is provided. The disclosure mitigates one or more bottlenecks in the development of in-situ extractive systems for production at scale, beyond laboratory testing.

In particular, the inventors realized that it would be desirable to provide a biocatalytic reactor including an upflow reaction section, a separation (product recovery/enrichment) section and a recycle flow section, whereby the separation section extends laterally away from an upflow reaction section of the reactor connecting to a recycle flow section therefrom back to the upflow reaction section. Inventors found that by extending the separation section laterally in a direction away from the upflow reaction together with a recycle flow section, the reactor and separation system allows for efficient recovery of a biocatalytically produced substance via an organic overlay. The efficient recovery advantageously allows keeping a concentration of a substance (e.g. produced reaction product in the reaction mixture) below a certain level. This can advantageously provide one or more of: improve stability of product and/or catalyst, enable pulling or pushing a equilibrium towards product formation, lower residence time of unstable or reactive products and/or reduce substance induced inhibition or toxicity.

Although a system or method according to the invention can suitably be employed at a pilot plant scale, demo plant scale or industrial scale, the system or method of the invention has been found particularly useful for process research or process development purposes. Thus, in a preferred embodiment the system is lab-equipment respectively the method is employed on a laboratory scale. The system respectively method of the invention is in particular advantageous in a laboratory environment in terms of one or more of: scale volume, efficiency, time and flexibility in operability, especially as regard to improving process conditions. One or more further or alternative objects of the invention which may be addressed follow from the remainder of the description.

Thus, the present invention provides an integrated multiphase biocatalytic reactor and separator system. The reactor and separator system are particular suited for continuous operation. The system may, however, also be used to advantage for non-continuous processes. For example, for semi-continuous, cyclic, or intermittent continuous operation, for batch or fed-batch operation, and/or as a chemostat. In particular, semi-continuous can be seen as any combination of batch and fed-batch operational windows of the different phases and streams combined in one fermentation protocol.

During operation, the reactor contents comprises at least two (phase-segregated) fluid phases. The first phase is a comparatively dense fluid phase (typically a liquid phase) and the second fluid phase is comparatively less dense (typically a gas phase). The difference in mixing ratio of lower density fluid and higher density fluid in the different sections creates a density difference between the sections. As result of this by gravitational forces a pressure differential exists between the sections creating a driving force for a fluid flow from one section to the other (such as a gas-lift effect). Thus, the inlet for the second fluid phase is advantageously arranged to provide a driving force for generating an upward flow in the reaction section.

The first fluid phase typically comprises one or more liquids, typically at least water, holding one or more biocatalysts. The biocatalyst can in principle be any biological material of biological moiety derived from a biological source having catalytic activity. The biocatalyst is, usually selected from enzymes (isolated from cells), microbial cells, cells from other organisms, cell extracts and the like; preferably cells are used, in particular living cells. The biocatalyst may be dissolved or dispersed (suspended). The first fluid phase, which may also be referred to as the biocatalytic liquid phase, generally also contains one or more substrates for biocatalytic conversion into a product (at least when present in the reaction section). Optionally the substrate may be fed as a separate phase. To separate (recover) formed product, a distinct third fluid phase (extraction or product recovery phase) having a density in between the first and second fluid phase. Typically the third fluid phase, i.e. product recovery phase, has a preferential affinity for the product over the biocatalytic liquid phase. The third fluid phase generally comprises a solvent for the product that is to be recovered, typically an organic solvent. Alternatively, or in addition, the formed product may segregate from the biocatalytic liquid phase, forming the third fluid phase on its own. In such case less or even no third fluid phase (such as an organic solvent for the produced substance of interest) may be required to recover the product from the biocatalytic liquid phase.

The reactor and separator system comprises one or more inlets for feeding reactor contents, an upflow reaction section in which reactor contents flow along a net upward direction (upflow), a separation section, and a recycle flow section. The upflow reaction section preferably comprises an inlet (typically a gas phase inlet), that is arranged to introduce the second fluid phase. Additionally the lowest density phase, may contribute to turbulent mixing of the reactor contents, optionally in combination with one or more mixing elements (e.g. a stirrer and/or a static mixer).

The separation section includes an outlet for the second fluid phase, typically a gas phase outlet, and the separation section extends in a lateral direction with respect to the net flow direction in the upflow reactor section and is configured to receive an effluent flow, i.e. lifted reaction contents, from the upflow reaction section, and includes a first separation zone and a second separation zone. During use the separation section provides a headspace (space above a fluid level) which is in fluid connection with the gas phase outlet

The first separation zone is configured for degassing the liquid effluent flow thereby providing a degassed effluent flow with reduced turbulence. The second separation zone is configured for liquid-liquid phase separation and is configured for receiving the degassed effluent flow from the first separation zone and to separate the degassed effluent flow, having reduced turbulence, in a lower liquid phase comprising a higher density liquid - typically comprising the biocatalyst - and an upper liquid phase having a lower density than the lower liquid phase, typically comprising one or more reaction products, and - if provided - the extraction or product recovery phase. The separation section comprises first separator outlet and a second separator outlet for respectively discharging the lower liquid phase and the upper liquid phase.

The recycle flow section comprises a recycle flow section inlet that is connected via a fluid passage way to the separation section outlet for receiving the lower liquid phase and a recycle flow section outlet opening into the upflow reaction section.

In some embodiments, the first and second separation zone can transition fluidly into each other. In a limit they could be seen as comprised in a single space.

During use the separation section zone may be conceptually seen as containing a mixture of phases having a gradually varying degrees of phase segregation (volume of intimately mixed phase divided by overall volume). Conceptually, a fraction of the phase with the lowest density (typically the gas phase) remaining within the effluent as received from the upflow reaction section progressively decreases. At the same time the relative fractions of the upper and lower density fluids progressively increase.

A benefit of the present invention is to enable or facilitate fermentation process development tailored to extractive overlay fermentation, in particular enabling or facilitating a (more) continuous fermentation process. The invention allows for increased production by removing inhibition/ toxicity and/or retaining longer cell viability resulting in longer fermentation duration and/or removal of unstable or reactive products increasing overall product yield and/or selection of strains most suited for more continuous extractive overlay fermentations and/or optimizing yield and specific product formation rate by the degree of freedom introduced by the device in the experimental set up of the fermentation. The reactor and separation system according to the present disclosure thus enables strain and process development to test: strain (genetic) stability over longer fermentation periods for continuous process at scale; test production titres, stability at longer fermentations. Further the system can be used to regulate/determine strain selection pressure.

The reactor and system according to the present disclosure can further enable large scale relevant process conditions as regard to screening and selection of strain(s), based on considerations like strains having a higher production activity of selectivity for longer period of time versus strain that can endure inhibition for a longer time or strains that can produce a high quantity of a product of interest in a short period (rate) before dying.

As detailed herein below, the reactor and separator system as disclosed herein can be further used to the advantage of producing and/or selecting one or more micro-organisms, e.g. from a library with a plurality of candidate biocatalysts in one or more cell cultures. For example, it is possible to select a suitable biocatalyst for the conversion of a particular substrate using a selection method, as described, herein below. Thus, in a specific aspect of the invention, the invention relates to a method of finding a biocatalyst capable of catalysing conversion of a particular substrate, comprising: providing the integrated reactor and separator system as disclosed herein with a library comprising a plurality of candidate biocatalysts in one or more cell cultures, which cultures comprise a culture medium containing a target substrate and/or one or more analogues thereof; selecting one or more candidate biocatalysts which grow in said culture medium; and screening for a biocatalyst which grows and/or proliferates in said culture having catalytic activity in converting the substrate. It will be appreciated that the reactor and separator system as disclosed herein can be of particular benefit over known systems, e.g. such as disclosed in WO2017/149099, on account of a comparative ease of in-situ controlling/adjusting a selection pressure during system operation, e.g. by offered flexibility in controlling one or more of residence times, operating temperature, and/or relative concentrations within the various reactor and separator zones.

As such the reactor can be employed for colonisation of microorganisms, e.g. for use in a upscaled reactor as disclosed in WO2021/010822.

Without wishing to be bound by theory inventors find that improved operating flexibility is at least in part provided by de-coupling of reaction conditions in the up- and recycle (or down) parts of the system, as enabled by the laterally extending liquid-liquid phase separation zone.

The reactor and system according to the present disclosure can further be used to test and select strains based on solvent interaction. The hydrophobic properties of microbial surfaces affect the affinity of a microbe for the hydrophilic fermentation environment, the hydrophobic solvent environment or the interface between these two. This behaviour can be an intrinsic property of the micro-organism but cell surface hydrophobicity can also be actively regulated. The current invention allows the straightforward selection and characterisation of microbes that preferentially reside in either the water or solvent phase. Further, it allows the assessment of cell behaviour during the full process time, thereby greatly reducing scale-up risks and control over process development.

Additionally, and as opposed to conventional overlay systems, the hydrolytic retention time of the product recovery phase/ phase comprising the product of interest can now be controlled in a reliable yet variable manner, e.g. by controlling an addition rate of a third fluid phase (e.g. a liquid organic phase for extraction of formed product) and/or a recovery rate of the phase comprising the product of interest at the separator outlet for the upper liquid phase . This can, for example, benefit control over specific fermentation behaviour in multi-phase liquid systems, like emulsion formation. For phase separation (product recovery) the use of a stable emulsion should be avoided. By controlling the third fluid phase, dosing and removal rate (and thus controlling third fluid phase hold-up in the system in general) the emulsion creating operational windows can be mitigated or essentially avoided altogether.

An additional benefit is that third fluid phase optimization (e.g. product removal by organic phase) can be studied in an ongoing fermentation process. Advantageously effects of third fluid phase dosing rate (and/or third fluid composition) and/or product concentration can be studied hand-in-hand with overall fermentation productivity. Advantageously, optimizing product removal/recovery can reduce downstream processing requirements (e.g. purification) either by stream size reduction and/or lower the number of unit operations required.

Additionally, biomass wash-out can be controlled as the liquid organic phase (e.g. as a third fluid phase) and biocatalytic liquid phase can be uncoupled. This also allows for lower wash out of biomass and the fermentation to go to higher cell densities, thicker fermentations.

The invention further provides a method for biocatalytic conversion of a substrate to a product. The method according to the invention comprises: flowing a reaction mixture comprising at least a liquid carrier, a substrate, and a biocatalyst upwards through the upflow reaction section of the reactor and separator system according to the present disclosure towards the separation section; providing a driving force for lifting the reaction mixture by feeding a gas phase into the upflow reaction section; receiving, at the first separation zone of the separation section including the gas phase outlet, an effluent flow from the upflow reaction zone; degassing the received effluent flow in the first separation zone thereby reducing a turbulence of the degassed effluent flow; and guiding the degassed effluent flow from the first separation zone along the second separation zone of the separation section, separating the degassed effluent flow into a lower liquid phase and an upper liquid phase, said lower liquid phase enriched in the liquid carrier compared to the degassed effluent, said upper liquid phase comprising enriched in formed reaction product, wherein the lower liquid phase is fed to the recycle flow section inlet of the recycle flow section of the integrated multiphase biocatalytic reactor and separator system via the first separator outlet, and wherein at least part of the upper liquid phase is discharged via the second separator outlet of the separation section; and recycling the lower liquid phase via the recycle flow section back to the upflow reaction section.

As such the method can be understood to comprise: flowing a reaction mixture comprising a first liquid phase comprising a substrate and a biocatalyst dissolved or suspended therein upwards through an upflow reaction section of a reactor and separator system according to the invention towards a separation section which separation section extends in a lateral direction with respect to a net flow direction in the upflow reactor section, by introducing a gas phase (typically providing a driving force lifting the reactor contents); receiving, at a first separator zone of the separation section including a gas phase outlet, an effluent flow from the upflow reaction zone, for degassing thereby reducing a turbulence of the degassed effluent flow; and guiding the degassed effluent flow from the first separator zone along a second separator zone of the separation section separating the degassed effluent flow (having reduced turbulence) in a lower liquid phase comprising the first phase liquid (typically comprising a water-based phase with biocatalyst (and typically remaining substrate, if any) and an upper liquid phase (a product recovery phase, typically comprising one or more reaction products, preferably in an organic carrier), wherein the lower liquid phase is fed to a recycle loop inlet of recycle loop of the integrated multiphase biocatalytic reactor and separator system via a first separator outlet, and wherein the upper liquid phase is discharged via a second separator outlet of the separation section; and recycling the lower liquid phase via the recycle loop back to the upflow reaction section.

### BRIEF DESCRIPTION OF DRAWINGS

These and other features, aspects, and advantages of the apparatus, systems and methods of the present disclosure will become better understood from the following description, appended claims, and accompanying drawing wherein:
FIG 1 provides a schematic cross-section side view of a reactor and separator system according to the invention;
FIG 2 provides a schematic cross-section side view of a further embodiment of the reactor and separator system during operation;
FIG 3A illustrates further aspects of the reactor and separator system during operation;
FIG 3B illustrates certain aspects at a top section of an embodiment of the reactor and separator system;
FIGs 4A and 4B illustrate further aspects at a top section of an embodiment of the reactor and separator system;
FIGs 5A and 5B provide schematic top and cross-section side views of further aspects of embodiments of the reactor and separator system.
FIGs 6A to 6D illustrate yet further aspects at a top section of the reactor and separator system;
FIG 7A and 7B provides schematic and detail cross-section side views of yet a further embodiment of the reactor and separator system;
FIG 7C provides a schematic cross-section side view of a further embodiment of the reactor and separator system;
FIG 8A schematically illustrates a for biocatalytic conversion of a substrate to a product;
FIG 8B and 8C depict photographs of embodiments of the reactor and separator system; and
FIG 9 and FIG 10 provide schematic cross-section side views of yet further embodiments of the reactor and separator system

### DESCRIPTION OF EMBODIMENTS

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise it will be understood that when a connection between structures or components is described, this connection may be established directly or through intermediate structures or components unless specified otherwise.

The invention is described more fully hereinafter with reference to the accompanying drawings, in which embodiments of the invention are shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout. Relative terms as well as derivatives thereof should be construed to refer to the orientation as then described or as shown in the drawing under discussion. These relative terms are for convenience of description and do not require that the system be constructed or operated in a particular orientation unless stated otherwise.

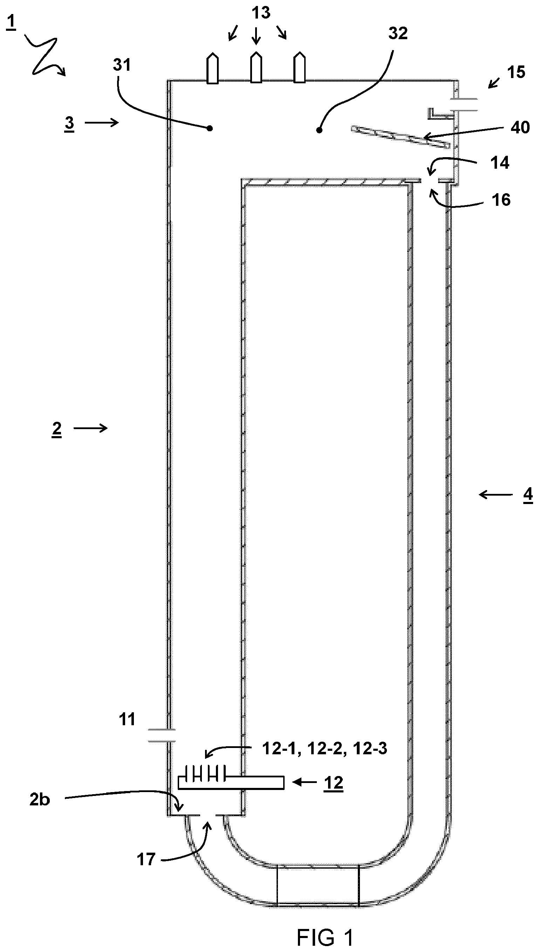

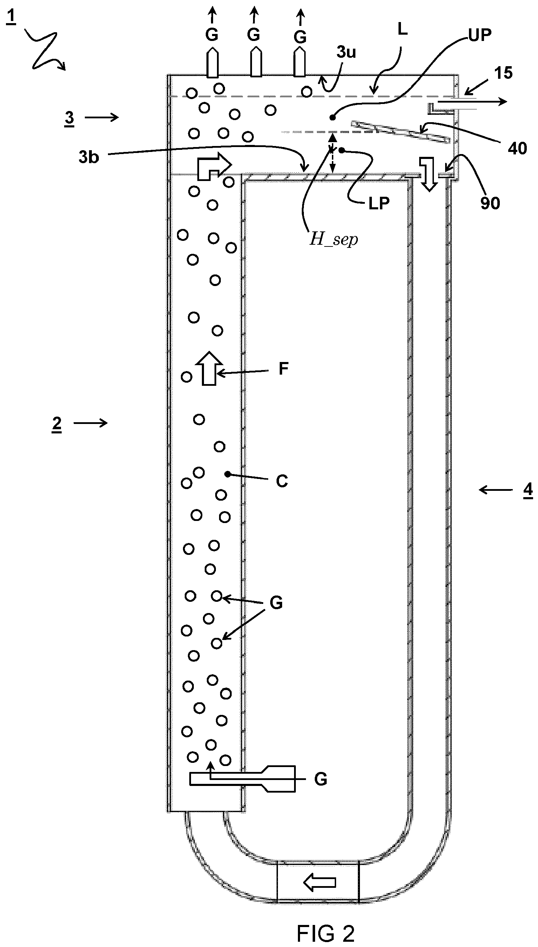

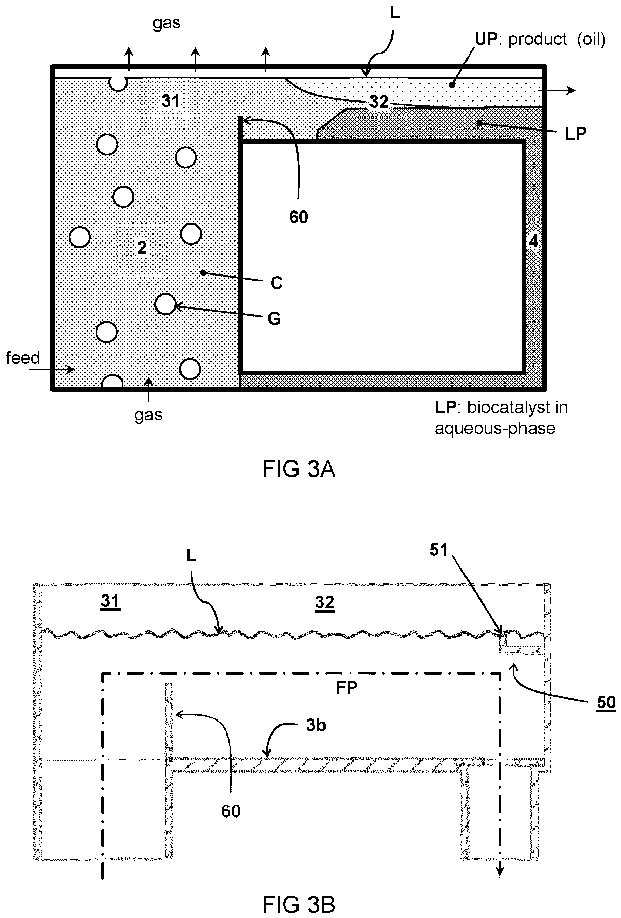

The invention and operating principles will now be explained with reference to FIGs 1, 2 and 3A, whereby FIG 1 provides a schematic cross-section side view of an embodiment of an integrated multiphase biocatalytic reactor and separator system according to the invention; and whereby FIGs 2 and 3A illustrate aspects of other or further embodiments of the reactor and separator system during operation.

As shown in FIGs 1, 2 and 3A, the reactor and separator system 1 comprises an upflow reaction section 2, a separation section 3, and a recycle flow section 4 that are interconnected, whereby the upflow reaction section 2 opens into the separation section 3, the separation section 3 opens into the recycle flow section 4 and the recycle flow section 4 opens back into the upflow reaction section 2, preferably at a bottom section thereof. As such the reactor and separator system 1 can be understood as defining a reactor for continuous circulation and separation of reactor contents C, e.g. as indicated in FIG 2. Of course the reactor and separator system can also be used in alternate modes of operation, including but not limited to non-continuous modes such as batch or batch-fed operation as mentioned herein above.

It will be appreciated that, as used herein, the reaction section 2, the separation section 3, form a single device or part of a single device, which may also be referred to as an integrated bioreactor, containing a reaction compartment and separate therefrom a separation compartment. The separation compartment advantageously allows separating and/or recovering the formed product from the catalytic phase. Advantageously, the integrated device may also include the recycle flow section 4 (e.g. for continuous operation). Alternatively, or in addition, the system may be connected to a storage for receiving recycled flow (e.g. for batch fed processes).

Advantageously the configurations as disclosed herein below allow routing the phase comprising the biocatalyst back to the reaction section under influence of gravity (height of the liquid column), without requiring a pump. Inventors found that solutions using a pump for driving the recirculation flow are less advantageous. Without wishing to be bound by theory inventors found that commercially available pumps negatively impart product separation performance as vibrations or limitations in pump performance can cause disturbances/variations in a liquid level and/or interface between phase segregated liquids within the separation section.

Having the recycle flow section 4 open into the upflow reaction section 2, preferably at a bottom thereof (e.g. as indicated in FIG 1 depicting a recycle flow section outlet 17 opening into a bottom-face 2b of the upflow reaction section 2 mitigates dead volume and/or stagnant areas within the system.

During operation, the reactor flow C comprises a biocatalyst either carried as a suspension or dissolved in a biocatalytic liquid phase, typically water. The biocatalyst being selected for biocatalytic conversion of a substrate to a desired product, typically an organic substance. In some embodiments, the organic substance and/or the substrate formed segregates from the biocatalytic liquid phase in a product recovery liquid phase that is largely immiscible with the biocatalytic liquid phase, i.e. forming a biphasic liquid-liquid phase separated system. In some preferred embodiments, a separate liquid phase is purposefully added to, mixed in, with reactor contents. The separate liquid phase selected to have a higher chemical affinity for the formed substance than the biocatalytic liquid phase. Adding a separate liquid phase reduces the concentration of formed substance(s) within the biocatalytic liquid phase (e.g. near the biocatalyst, i.e. cell membrane in case of living organism). Removing product results in lower concentration in the biocatalytic liquid phase (e.g. near cell membrane), removing or alleviating inhibition and/or improving conversion rate of the biocatalytic reaction, particularly for an equilibrium reaction. Depending on the biocatalytic reaction process the reactor contents may include one or more further constitutes, e.g. additives such to sustain a living biocatalyst (e.g. a yeast, for instance *S. cerevisiae*; bacteria, for instance *E. coli*) such as salts, vitamins, other nutrients, pH adjusting agents, surfactants/antifoams and/or a marker (e.g. a fluorescent marker) to monitor a mixing condition and/or activity of the biocatalyst.

The term biocatalytic liquid phase as used herein is used to indicate the phase holding the biocatalyst, i.e. the liquid phase that carries the biocatalyst (e.g. a suspended yeast or a dissolved enzyme). This phase is herein also sometime referred to as 'first liquid phase' so as to distinguish the phase over further phases, such as the second liquid phase (i.e. the third fluid phase), which as used herein generally refers to a phase having a comparatively lower density, e.g. an organic phase, which comprises or can even be essentially composed of converted substrate and/or the optional extraction phase. Likewise it will be understood that the terms upper and lower liquid phases, as formed from the lifted reactor content in the phase segregation section 3 are used to indicated the relative position of the respective segregated phases, i.e. the upper phase being the comparatively lower density phase that is enriched in formed reaction product and optionally the second liquid as compared to degassed effluent from the upflow reaction section, and the lower phase being the comparatively denser phase which is enriched in the liquid carrier as compared to the degassed effluent.

It will be understood that the reactor and separator system 1 comprises one or more inlets 11 for feeding constituents of the reactor flow C, including one or more for the biocatalytic liquid phase, the product recovery liquid phase, the biocatalyst, one or more substrates, carrier liquid, and additives. In principle all constituents may be added via a single inlet. However, to avoid cross-contamination and allow individual rates of addition the system preferably comprises a plurality of feed inlets, e.g. separate inlets for the first fluid phase and one or more of the further constituents or inlets for mixtures of the constituents. Said inlet or inlets may be suitably positioned to provide a feed entry at any point along the system, e.g. the recycle flow section. Preferably, the inlet is positioned at the bottom section of the upflow reaction section 2. Feeding the reactor at its bottom portion, e.g. as shown in FIG 1, or even at the bottom 2b of the upflow reaction section 2, can advantageously improve the biocatalytic reaction, e.g. in terms of an overall conversion of substrate by the biocatalyst. Typically, the reaction section extends for at least 25 cm. Shorter columns are possible but have less driving force. Providing the gas inlet near or at the bottom section further optimizes driving force for recirculation, mixing of reactor constituents, while minimizing a dead volume within the upflow reaction section 2. Also a stirring element can be placed to further enhance mixing.

The integrated multiphase biocatalytic reactor and separator system 1 further comprises a gas phase inlet 12, which gas phase inlet is arranged to introduce a gas phase G into the upflow reaction section 2. During operation the mixing of gas phase G with the contents of the reaction section creates a density difference between the sections, thereby creating a driving force for a reactor flow C in the upflow reaction section. The gas phase inlet 12 is typically provided at a bottom section of the upflow reaction section 2, preferably near the bottom-face 2b. It will be understood that gas can alternatively or in addition be fed from a top section of the upflow reaction section 2, e.g. in the form of a sparger extending down in the reaction compartment. The gas to be used can be chosen dependent on known considerations for the type of biocatalyst to be used and the product to be produced. E.g. when aerobic conditions are desired oxygen or an oxygen-containing gas (such as air) is usually used. For anaerobic conditions nitrogen is particularly suitable. In some embodiments a substrate for the biocatalyst is a gaseous and can thus be included as (part of) the gas phase.

The gas inlet typically comprises a plurality of distributed nozzles 12-1, 12-2, 12-3, e.g. a sparger, opening into the upflow reaction section 2 so as to, in use, provide a spatially distributed stream of gas bubbles and influence gas bubble size by choice of specific nozzle size or type and/or mechanical stirring. Also, in case of aerobic reactions, smaller bubbles increase higher surface area and improve gas (e.g. O₂, CO₂) transfer to/from the phase with the biocatalyst. In a preferred embodiment, the gas phase inlet 12 is operatively controlled by a controller allowing dynamic control over a gas flow rate entering the upflow reaction section 2. Dynamic control of the gas flow rate and nozzle configuration creates control of the gas bubble size and distribution and thereby allows control over the driving force for recirculation, optionally aided by mechanical stirring.

As shown in, e.g. in FIGs 1 and 2, the upflow reaction section 2 opens into a separation section 3 which is configured to receive an effluent flow, i.e. lifted reactor flow, from the upflow reaction section and includes a first separation zone 31 configured primarily for degassing the effluent and a second separation zone 32, configured for receiving the degassed effluent flow from the first separation zone, and which is configured to separate the degassed (ef)fluent flow, having reduced turbulence as received from the first separation zone, into a lower liquid phase 'LP' and an upper liquid phase 'UP'. The lower liquid phase 'LP' typically comprises the biocatalytic liquid phase. In some embodiments, the lower liquid phase (LP) advantageously comprises one or more of the biocatalyst, unconsumed substrate, for recovery or further processing after recirculation and/or one or more of the additives, if present. The upper liquid phase 'UP', typically comprises the product recovery liquid phase, e.g. produced reaction substance and the solvent if present. The upper liquid phase (UP) can advantageously also comprise other components such as inhibiting components, so that these can be removed from a running process cycle. The upflow reaction section 2 is generally configured to be positioned, at least during operation, in an upright orientation, preferably with a deviation smaller than 20 degrees. Orienting the upflow reaction section 2 vertically mitigates preferential flow of the gas phase along upstanding bounds of the upflow reaction section, improves gas hold-up and improves gas distribution in the upflow reaction section.

Importantly, and in contrast to known multiphase biocatalytic reactors, the separation section 3 in the reactor and separator system 1 according to the invention is configured so that it extends in a lateral direction with respect to net flow direction F in the upflow reaction section 2, whereby degassing is accomplished without needing a down-comer for degassing.

In an advantageous embodiment the separation section extends in a lateral direction relative to the upflow reaction section beyond the width of the reaction section. At least in such embodiment, the recycle flow section generally comprises an external loop wherein the outlet for the fluid to be recycled from the separation section to the reaction section is spatially separated from the inlet for the recycle flow into the reaction section.

Obviating a need for a down-comer, as typically required in the art, advantageously enables the use of a comparatively larger fraction, or even the complete, potential energy provided by the height of the liquid column as driving force for the recycling back the lower liquid phase LP. As such the system can advantageously be operated without a need for a displacement pump. Omitting, a displacement pump further eliminates pump-induced factors (e.g. vibrations or liquid level variations inside the separation section) that negatively impart liquid/liquid phase segregation.

The dimension of the separation section 3, e.g. length and/or compartment size can be adjusted e.g. by replacing a separation section 3 for a comparatively longer separation section 3 to adjust residence time and/or flow regime and accordingly improve separation. Adjustment of liquid height in the separation section can also be used to improve separation. A larger height can be applied to contribute to a reduced flow rate, reduced turbulence, and increased residence time. As such dimensioning of the separation section 3 advantageously provides a degree of freedom for reactor design and/or process development.

The first separation zone 31, which is positioned above the upflow reaction section 2 typically includes one or more gas phase outlets 13 for discharging gas phase G released from the reaction mixture thereby reducing a turbulence of the lifted reactor flow C as the gas (which may include gas produced in the reaction section) is released and increases density of the remaining reaction mixture contents. The gas outlet is generally provided above a target liquid level L, e.g. in an upper bound 3u of the separation section 3. The target liquid level L is preferably set such that there is sufficient liquid hold up in the system to create a continuous recirculation flow.

The second separation zone extends in a side-ways direction forming a conduit section extending from the first separation zone along a base 3b of the separation section 3 towards a first separator outlet 14 providing a flow path for settling the received reactor effluent as it is conveyed gradually and progressively reducing turbulence towards an essentially laminar flow, allowing the degassed reactor effluent to progressively segregate into an upper liquid phase 'UP' and a separate lower liquid phase 'LP' e.g. as schematically indicated in cross-section side view in FIG 3A. As such the second separation zone 32 can be understood as being a liquid-liquid phase separator. The lower liquid phase 'LP' comprises-the biocatalyst, the first liquid phase, unreacted substrate (if present), and one or more of the additives (if present). The upper liquid phase 'UP' typically comprises produced reaction substance and - if a solvent is used for product recovery is used - the solvent liquid for the recovered product.

The lower liquid phase 'LP' is discharged from the separation section 3 via a first separator outlet 14 positioned near, preferably at, the base 3b to an inlet 16 of the recycle flow section for recycling back to the upflow reaction section 2. At least part of the upper liquid phase 'UP', containing the formed substance, is discharged from the system via a second separator outlet 15 that is positioned above the first separator outlet 14 at a height corresponding to a level of the upper liquid phase, having a lower density. A remainder of the upper liquid phase (e.g. <10% or <20%) may be recycled along with the lower liquid phase 'LP' towards the upflow reaction section 2. Recycling part of the upper liquid phase (UP) improves product recovery, product purity and/or mitigates loss of biocatalyst from the system via the recovered product stream. The interface between 'LP' and 'UP' (mass balance between LP and UP) relates to the hold up of 'LP' and can be chosen by controlling the in and out flows. Preferably the interface is controlled at a constant level and set such that the turbulence in the second separation zone 32 is reduced such that it allows and promotes liquid-liquid separation.

Preferably, the second separator outlet 15 is positioned above an equilibrium phase boundary between the lower and upper liquid phase. Preferably second separator outlet 15 is submerged below target liquid level L so no gas entrains with the upper liquid phase outlet (see e.g. FIGs 2 and 3A). Positioning the second separator outlet 15 well above the interface level of the lower and upper liquid phase, preferably as high as possible, minimizes concentrations of unreacted substrate, lower liquid phase and/or biocatalyst in the product recovery liquid phase flow, thereby maximizing the fraction of 'UP' in the separator outlet 15 flow and mitigating loss of biocatalyst, as the function of the second separator outlet 15 is to preferably remove the upper liquid phase from the system.

The recycle flow section 4 comprises a recycle flow section inlet 16 that is connected to the first separator outlet 14 for the lower liquid phase and a recycle flow section outlet 17 opening into the upflow reaction section 2. The recycle flow section inlet is usually positioned higher than the said recycle flow section outlet. Positioning the recycle flow section outlet below the recycle flow section inlet generates a nett positive driving force for recirculation. Having a net positive driving force advantageously allows back feeding the recycle flow without a use of dedicated liquid displacement means such as pumps, i.e. driving the recycle flow exclusively by provision of gravitational force as result of density difference between the different sections (hydrostatic pressure). A positive driving force can be provided by assuring a value larger than zero for a hydrostatic pressure differential between the contents in the reaction section and in the recycle flow section minus friction losses.

In a preferred embodiment, e.g. as shown in FIG 1 and 2, the reactor and separator system 1 includes a flow guidance member 40, typically a panel. The flow guidance panel 40 extends within the separation section 3, typically within a downstream portion of the second separation zone 32 to guide the lower liquid phase 'LP' flow towards the first separator outlet 14. As shown said member can be understood to provide an impact plane across a flow path for the degassed reactor effluent at a position across a phase boundary between the upper liquid phase 'UP' and the lower liquid phase 'LP', said phase boundary schematically illustrated in FIG 2 by a dashed line between the upper liquid phase 'UP' and the lower liquid phase 'LP'. Said member providing an impact plane can be tilted, slanted or more complexed shaped, as long as it promotes the desired flow behaviour (in relation to turbulence) to allow or enhance upper liquid phase separation. It will be understood that the phase represented as a plane can in practice actually be a transition zone having a gradual transition between opposing phases with respective droplets of a foreign phase in each phase gradually coalescing and separating out as the flow progresses along the flow path. Provision of the guidance member can advantageously improve a purity of the upper liquid phase 'UP' by directing a portion of the upper liquid phase 'UP' at the transition zone towards the first separator outlet 14.

In other or further embodiments, e.g. as show in FIGs 1, 2, and 3B, the reactor and separator system comprises an overflow element 50. The overflow element is positioned at the second separation zone 32 directly upstream of the second separator outlet 15 and configured to restrict passage of liquid (the upper liquid phase UP) towards the second separator outlet 15 at a liquid level L exceeding an overflow boundary 51 of the 50 overflow element. Providing an overflow element advantageously further reduces a fraction of lower liquid phase (LP) in the collected product flow by limiting the collected fraction to an uppermost portion of upper liquid phase 'UP' and recirculating the remainder, e.g. for further conversion. In a preferred embodiment, the overflow element 50 is equipped with a drain hole to drain lower liquid phase in case it gets in there, e.g. by splashing of liquid from the section first separation zone 31 to the second separation zone 32.

In some particularly preferred embodiments, e.g. as shown in FIG 3A, the reactor and separator system further comprises a partial barrier 60, e.g. baffle, extending upwardly within the separation section 3 from a base 3b of separation section 3. The baffle can be understood as forming a partial barrier within the separation section 3 barrier defining a transition between the first and the second separation zones 31,32. The barrier 60 can advantageously prevent a flow of degassed reactor effluent along a back direction towards the upflow reaction section 2. The barrier 60 contributes to reducing turbulence in the flow transition zone from the reaction section to the separation section. Of course an equilibrium liquid level L, e.g. as illustrated in FIG 3A and 3b can be higher than the barrier, by regulating liquid (and/or gas) hold up in the system. Higher gas hold up levels will increase the driving force for recirculation and vice versa. The liquid level can be controlled, tuned, e.g. by regulating one or more of a feed rate of the reactor flow C, the driving force for turbulently mixing and for lifting the reactor flow, and the exit rate at one or more of the first and second separator outlets 14,15.

Inventors found that an exit flowrate at the first separator outlet 14 and/or the second separator outlet 15 can be suitably controlled by one or more removable or adjustable further restriction elements. Accordingly, in a preferred embodiment, the reactor and separator system comprising one or more removable or adjustable further restriction elements 90 to reduce a flowrate of the lower liquid phase 'LP' toward the recycle flow section 4. Or in other words, the restriction element 90 can be used to be able to maintain a particular gas hold up in the reaction section, e.g. in cases where a higher gas hold up is needed for reaction but a lower recirculation flow for separation, by reducing the driving force for recirculation by restricting the flow towards the recycle flow section 4 resulting in increased residence time for separation. The restriction elements 90 can for example comprise one or more reversibly mountable and/or adjustable elements such as washers or orifice. Optionally, the restriction elements 90 are adjustable flow regulation means, e.g. an adjustable diaphragm, and/or a programmable restriction valve. It will be understood appreciated that the restriction element, if present, need not necessarily be provided at an entrance of the recycle flow section 4. Alternative positions having an effect on the driving force for recirculation are equally possible, e.g. more towards the recycle flow section outlet 17.

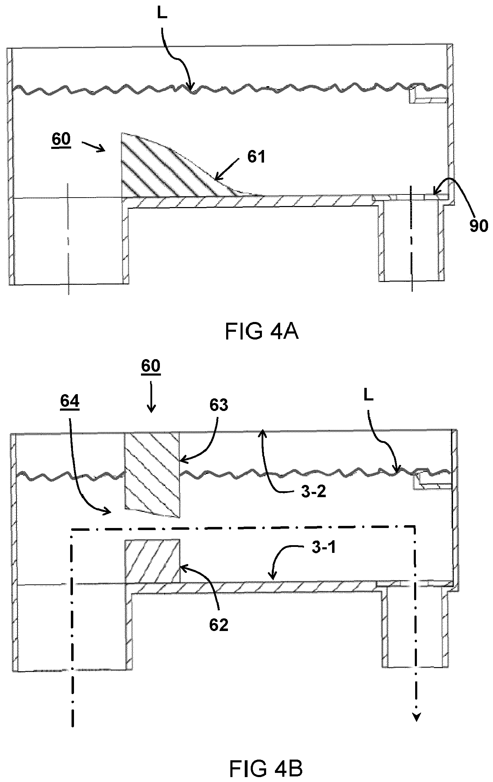

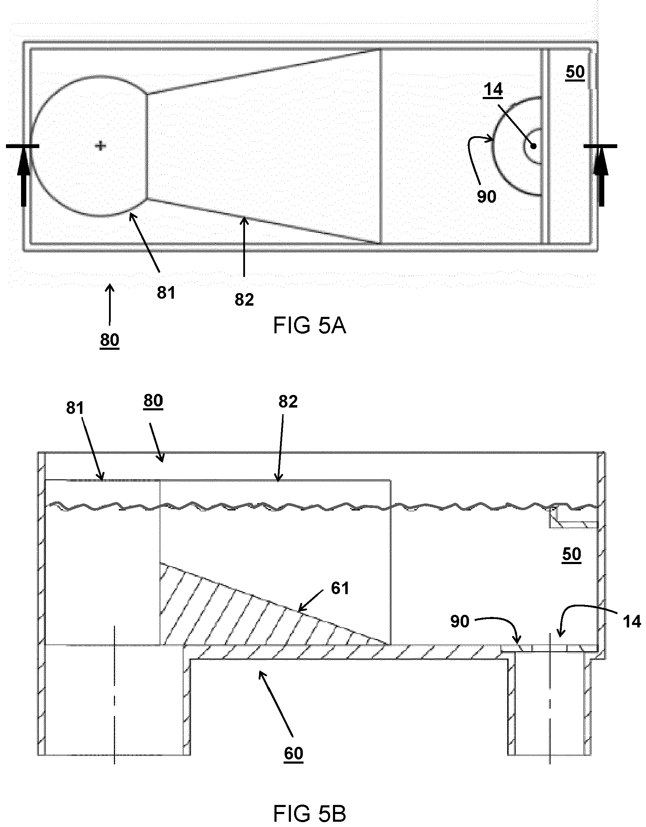

Returning back to the barrier 60 further advantageous embodiments will now be detailed with reference to FIGs 4A, 4B, 5A and 5B.

In an embodiment, e.g. as shown in FIG 4A, the barrier includes a downstream portion 61 that gradually slopes toward the base 3b. Providing a gradually downward sloping portion 61 advantageously smoothens out a flow profile downstream of the barrier, reducing turbulence, e.g. Eddies, in the wake of the barrier. Mitigating turbulence within the second separation zone 32 advantageously improves phase separation between the first and second liquid phases and therefore purity of the flows at separator outlets 14,15.

In another or further embodiment barrier 60 is configured in a slit 64 configuration including lower section 62 extending from a central the slit to the base 3b and an upper section 63 extending from the slit to an upper bound 3u of the separation section 3. Arranging the barrier in a slit configuration advantageously forces a comparatively larger portion of the gas phase as comprised in the lifted reactor flow C to be released upstream of the barrier 60, i.e. within the first separation zone, thereby improving liquid-liquid separation downstream of the barrier, e.g. by mitigating disturbances at a liquid-gas interface L by bubbles separating out within the second separation zone. Arranging the barrier in a slit configuration advantageously further prevents splashing of liquid from section 31 onto liquid level L in section 32

In yet further embodiments, e.g. as shown in FIGs 5A&B, the reactor and separator system comprises a flow guiding element 80 that is positioned at an interface between the upflow reaction section 2 and the separation section 3.

As shown in top-view the flow guiding element 80 includes a first flow guiding element 81 and second flow guiding element 82 connected to the first for respectively receiving a lifted reactor flow and diverting the received flow towards the second separation zone 32. As shown in FIGs 5A and 5B the first flow guiding element comprises a volume having a reduced cross-sectional area at a longitudinal position above the upflow reaction section. The second flow guiding element 82 defines a second volume that extends laterally from the first and which has a gradually increasing cross-sectional area to open into the second separation zone (32).

The first flow guiding element 81 effectively reduces a volume of the first separation zone 31 wherein the received reactor flow is at least partly degassed. From the first flow guiding element 81 the at least partially degassed reactor flow is diverted laterally to a volume defined by the second flow guiding element 82. The second volume has a gradually increasing cross-sectional area along a lateral direction that opens into the second separation zone. The flow guiding element 80 reduces an apparent volume within the separation section 3, which can be used to control or regulate a residence time and/or liquid level within the separation section 3. Inventors found that provision of the flow guiding element 80 can further advantageously improve phase separation, whereby the comparatively restricted diameter (relative to the upflow reaction section 2) in the first flow guiding element 81 improves release of gas phase by inducing a local increase in flow rate and the second flow guiding element improves a liquid-liquid phase separation by promotion of a more laminar flow profile due to an induced decrease in flow rate.

Advantageously the flow guiding element 80 can be integrated with the barrier 60 as describe above for combined benefits. In a preferred embodiment, the barrier 60 includes a gradually downward sloping portion 61 which further builds upon reducing a flow speed towards the second separation zone 32 while mitigating disturbances due to Eddies, e.g. as shown FIG 5B depicting a linearly sloping portion 61.

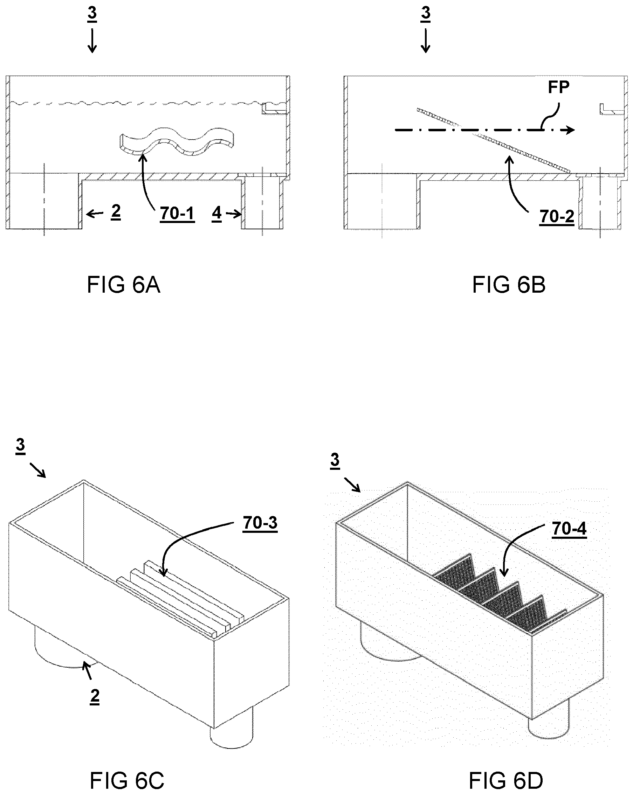

To further control liquid-liquid phase separation the embodiments as disclosed herein can advantageously be provided with one or more coalescence promoting elements. These elements extend within the second separation zone, upstream the first and second separator outlet. As will be explained with reference to FIGs 6A-6D these elements 70 advantageously promote liquid-liquid phase separation. Such elements stimulate coalescence of dispersed droplets comprising the produced product of interest. This can for instance be achieved by providing the surface of a elements with a coalescence-promoting material. Without wishing to be bound by theory inventors find that the coalescence promoting elements improve liquid-liquid phase separation by providing impact areas that promote coalesce of micro-phase separated domains into progressively larger phase domains (droplets).

In an embodiment, e.g. as illustrated in FIG 6A, the coalescence element 70-1 is configured as a plate extending longitudinally along an overall flow direction within the separation section 3 having a plurality of protrusions or undulations, e.g. a wavy plate, that provide impact surfaces thereby promoting coalescence of upper phase liquid.

In another embodiment, e.g. as shown in FIG 6B and 6D, the coalescence element 70-2 is configured as a microperforated plate or mesh oriented. The perforated panel 70-2 is preferably positioned in a downward slanted orientation across the flow path FP.

Alternatively, or in addition, the system may be provided with a plurality of parallel oriented coalescence elements 70, e.g. as shown in FIG 6C depicting a plurality of vertically oriented plates 70-3. The plates 70-3 preferably are at an angle of 15° to 70°, more preferably an angle of 30° to 45°.

As a further alternative it is envisioned to provide the system with a plurality of microperforated (e.g. a mesh) members 70-4 positioned in series along an overall flow direction within the separation section 3, preferably having progressively increasing pore sizes towards the outlets so as to promote coalescence in progressive larger phase separated domains.

In some embodiments, the one or more of the flow guidance panel 40, the barrier 60, the coalescence element 70, and/or sidewalls of the separation section 3 are coated with a coalescence promoting material, such as a material having lipophilic/hydrophobic qualities. Alternatively the flow guidance panel 40, the barrier 60, the coalescence member 70 may comprise, or essentially consist of, a coalescence promoting material. Alternatively, or in addition, coalescence promoting properties may be provided by a surface relief pattern of the part (lotus effect). Coalescence promoting material configured to promote liquid-liquid phase segregation may be provided by non-stick or low surface low surface energy coatings (below 36 dynes/cm), such as non-fouling coatings or fluoropolymer based coatings. Suitability of materials/surface patterns may be conveniently determined by method known in the field, such as contact angle measurements. For example, for a water-based catalytic system yielding a lipophilic product, a suitable coalescence promoting material may be a material, coating, and/or surface relief displaying a low contact angle for the product phase (e.g. < 20 degrees) and a high contact angle for the water phase (e.g. product > 90 degrees). As an example, inventors found that Tygon (Saint Gobain) provides favourable surface interactions with a bio-based oils

It will be appreciated that the design of the present reactor and separator system 1 allows for a modular assembly, enabling incorporation, adjusting, replacing, or removing one or more of one or more of the flow guidance panel 40, the overflow element 50, the barrier 60, the coalescence element 70, the flow guiding element 80 and/or flow restrictor 90. Thereby allowing versatile control over one or more of the flow rate, residence time, a hydrostatic pressure, and a separation efficiency, within the system without requiring a complete redesign, overhaul, or modifications to reactor dimensioning, in particular the upflow reaction section 2, the separation section 3, and the recycle flow section 4. As such the system as disclosed herein can be of suitably adjusted to the needs of a particular biocatalyst or reaction conditions and/or for testing new biocatalysts and/or process conditions.

Further it will be appreciated that the system can advantageously be equipped with one or more of a sensor e.g. to measure reaction conditions, an impeller (motor) for providing mixing/dispersion of a feed flow or lift gas and/or reactor flow constituents, a heating and/or cooling means, and a monitor, preferably in combination with a control system configured to adjust one or more process conditions in dependence of a measured output from the one or more sensors. The sensors can include one or more of a temperature sensor, a pH sensor, and an oxygen sensor, respectively configured to measure a temperature of the reaction mixture, an pH of the reaction mixture, and an oxygen concentration in the reaction mixture and or gas phase. Advantageously the system can further comprise a monitor/control system configured to adjust one or more of a temperature, lift forces, and/or a feed rate of one or more of the constituents comprised in the reaction mixture including additives such as a pH adjusting agent, in dependence of a measured output of the one or more sensors and/or a predetermined control parameter, such as upper or lower temperature of pH limits. Provision of a monitor/control system in combination with the one or more sensors, e.g. an off-gas analyser, can advantageously improve an output and or lifetime for certain biocatalysts.

In a preferred embodiment, at least part of the walls bounding the one or more of the upflow reaction section 2, the separation section 3, and the recycle flow section 4 are optically transparent. Forming at least part of the system walls of an optically transparent composition (e.g. glass or polycarbonate) allow visual inspection of one or more of a liquid fill level, a progression of degassing, and progression of liquid-liquid phase separation (e.g. within the second separation zone).

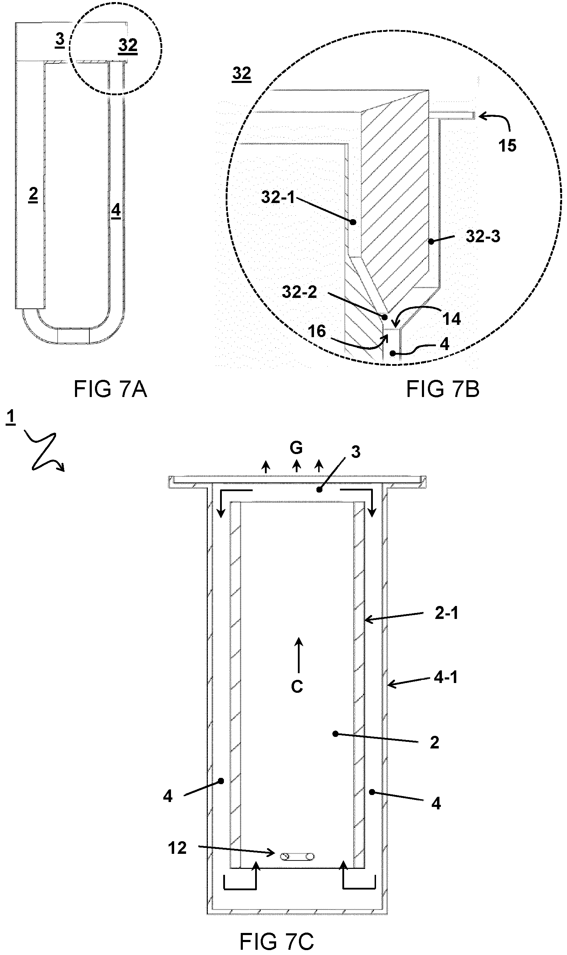

In some embodiments, e.g. as shown in FIG 7A, the liquid-liquid phase separator can advantageously be arranged to include a conduit section 32-1 configured for diverting a received degassed effluent flow downward towards a diverging connection member 32-2. Said diverging connection member is connected the recycle flow section via the separator outlet 14 and to the second separator outlet 15 via an upward conduit section. Both of the upward conduit section 32-3 and the connection to the recycle flow section 4 via the second separator outlet 15 having a comparatively larger cross-sectional diameter (32-2 and 14/16) to reduce a local flowrate of degassed effluent flow thereby providing liquid-liquid phase separation in an upper liquid phase (diverted to the second separator outlet) and a comparatively heavier lower liquid phase (recycled back to the upflow reaction section 2 via recycle flow section 4).

In some embodiments, e.g. as shown in FIG 7C, the reactor and separator system is arranged in core-shell configuration comprising an inner tube section 2-1 defining the upflow reaction section 2, surrounded by an outer annulus 4-1 defining the recycle flow section 4. As for the systems depicted in FIGs 1,2 and 3 the core-shell design can advantageously be used for continuous operation , e.g. in biocatalytic conversion of a substrate to a product, whereby a reactor flow C is guided (lifted) across a upflow reaction section towards a separation section 3 for degassing and liquid-liquid phase separation into an upper liquid phase and a lower liquid phase, which lower liquid phase is recycled back towards the upflow reaction section. The core-shell configuration, e.g. as depicted, advantageously offers a comparative smaller form-factor and/or increased production rate per volume unit reactor space. Note that, for ease of understanding, not all features discussed in relation to FIGSs 1 to 7A are depicted. It will however be understood that such features can be used to advantage with the core-shell configuration unless excluded by context.

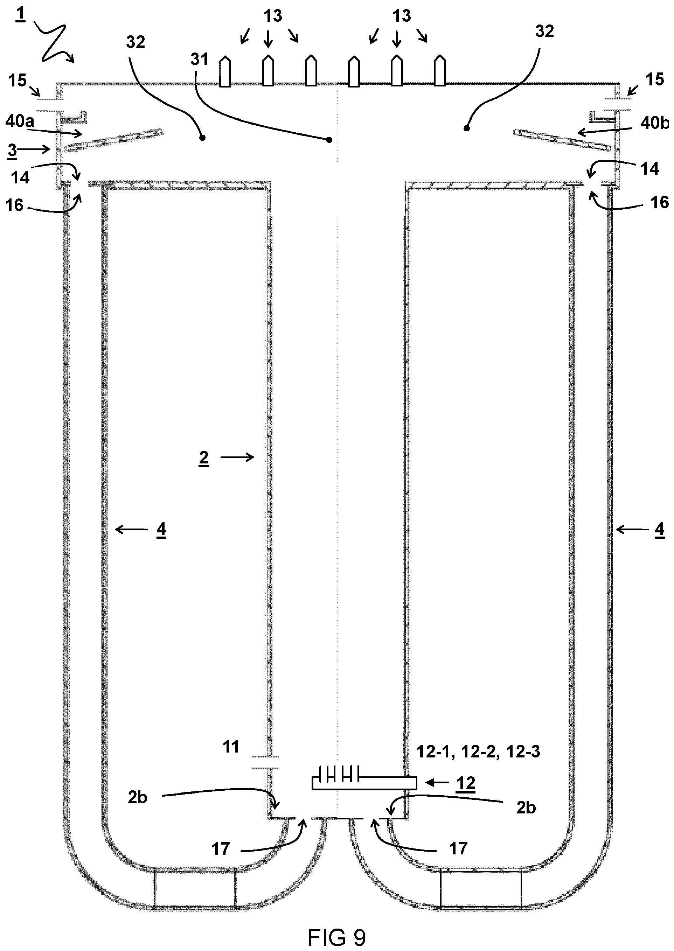

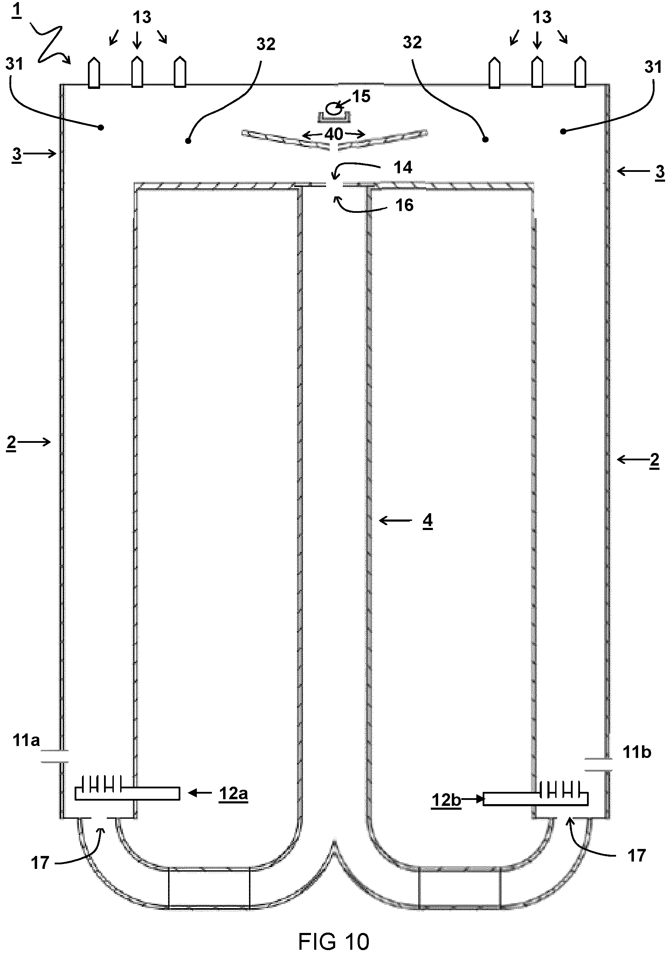

Referring forward, FIGs 9 and 10 illustrate yet further embodiments of the integrated multiphase biocatalytic reactor and separator system 1 as disclosed herein.

The reactor and separation system as shown in FIG 9 differs from systems as described in relation to FIG 1-3 in that the upflow reaction is flanked by two recycle flow sections 4 fed by individual separation sections 3. Providing the reactor and separation system with a plurality of recycle flow sections can increase an overall production rate for a given footprint. Additionally provision of a plurality of separation sections and recycle loops in situ evaluation the effect on process operation of different elements such as flow guidance panels 40a and 40b and/or evaluation the effect of different coalescence members, e.g. as shown in FIG 6A-B. If one needs more separation capacity versus reaction capacity, splitting the flow into two or more recycle section will decrease the recirculation rate.

It is also possible to provide two or more reaction sections. This can be used to increase reaction capacity versus separation capacity, as having more reaction sections will increase the recirculation flow and capacity versus separation. The reactor and separator system as shown in FIG 10 differs from systems as described in relation to FIG 1-3 in that it comprises a plurality of upflow reaction sections 2 flanking a central recycle flow section 4. Similar to the embodiment shown in FIG 9 configurations comprising a plurality of upflow reaction sections can increase a reactor yield for a given footprint. Alternatively, or in addition, configurations comprising plurality of upflow reaction sections can be used to advantage to evaluate the effect of different process conditions (e.g. gas flow rate and/or feed rate from inlets 11a,11b, and 12a, 12b).

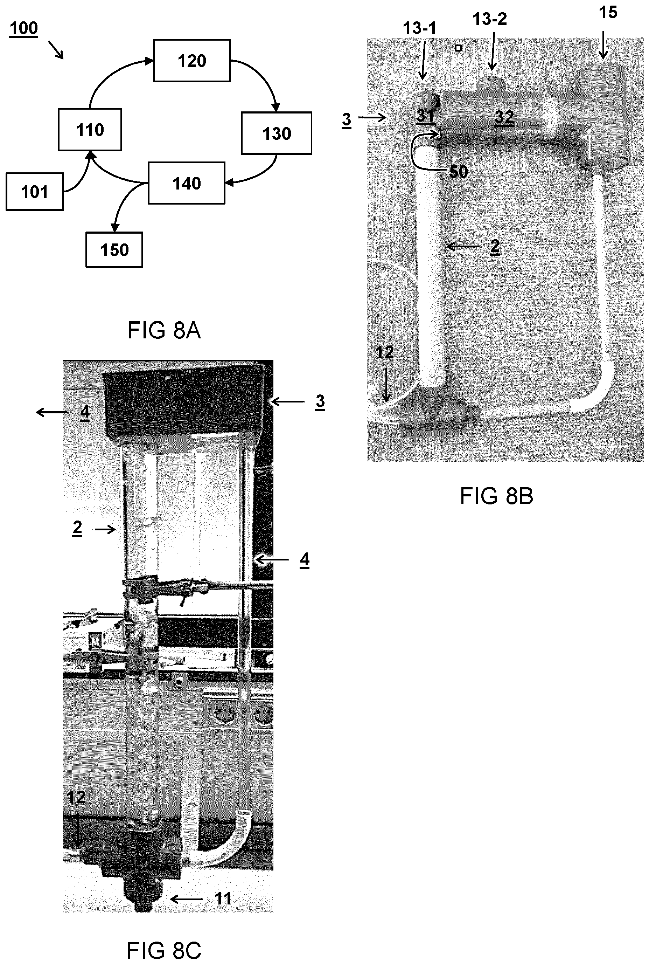

Returning back to, FIG 8B and 8C display photographs of two exemplary modularly assembled embodiments of the reactor and separator system according to the invention.

The embodiment shown in FIG 8B includes a separation section 3 that is connected essentially directly above the upflow reaction section 2 for receiving and at least partly degassing a lifted effluent flown therefrom. Released gas can escape via a first gas phase outlet 13-1. The first section is connected to a cylindrical pipe section (forming the second separation zone 32) that extends in a lateral direction from the upflow reaction section 2 (radially) to allow degreased liquid to settle in a upper liquid phase 'UP' and a lower liquid phase 'LP'. A further gas phase outlet 13-2 is provided to allow residual gas to escape the system. The cylindrical pipe section is connected at a distal end to a separator outlet 15 for collecting the upper liquid phase and to a recycle loop 4 configured to recirculate the lower liquid phase back towards the bottom of the upflow reaction section 2. The barrier 60 in this embodiment is provided by a terminal wall section of the cylindrical second separation zone 32 at its interconnection to the first separation zone 31.

The embodiment shown in FIG 8C is shown during operation. The embodiment differs from the embodiment in FIG 8B in that the upflow reaction section 2 and the recycle flow section 4 are at least partially formed of an optically transparent composition allowing visual inspection of respective process flows. The embodiment further differs in that the separation section 3 in this embodiment is composed of a rectangular compartment having an open top to allow gas to escape and for product collection. Also shown in FIG 8C is an inlet 11 for feeding first and second phase constituents to the reactor.

As shown, various components, including one or more to the upflow reaction section 2, the first separation zone 31, the second separation zone 32 and the recycle flow section 4 are configured as modular elements which, in contrast to a large scale system, can be replaced or exchanged comparatively easily to evaluate different processes and/or process conditions.

Inventors confirmed that for both configurations a continuous circulation flow can be sustained by a driving force as provided by an upward lift from gas provided from inlet 12 without a need for additional fluid displacement means, even with gas flow rate at a minimal setting.

Alternatively, or in addition, the gas phase can also be provided from a sparger, e.g. a sparger extending down in the reaction compartment from an inlet at an upper portion upflow reaction section.

Inventors further confirmed that is possible to ascertain a near to 100% (>95%) efficiency for both degassing (gas-liquid phase separation) and liquid-liquid phase separation for a number of reaction mixtures for various binary solvent combinations including water-based and oil-based compositions over a broad range of densities and viscosities such oleyl alcohol (~0.85 g/cm³; 28 mPa.s) sunflower oil (~0.92 g/cm³; 23 mPa.s) and castor oil (~0.95 g/cm³; 450 mPa.s). Inventors further confirmed that steady state separation efficiency for all different solvent mixtures was excellent (<5 vol% of water phase in collected oil phase) was essentially unaffected from oil phase properties, airflow setpoints, and oil inflow rates.

As mentioned the integrated multiphase biocatalytic reactor and separator system 1 as disclosed herein can be used for biocatalytic conversion of a substrate to a product and recovering the product from the reaction mixture. FIG 8A schematically illustrates a method 100 for biocatalytic conversion of a substrate to a product. The method 100 according to the invention comprises: directing 110 a reaction mixture comprising a first phase liquid (typically comprising a water-based phase) with biocatalyst and substrate, upwards through an upflow reaction section of a reactor and separator system as disclosed herein towards the separation section 3 which separation section extends in laterally with respect to net flow direction in the upflow reactor section, by introducing a gas phase G; receiving 120, at a first separation zone 31 of the separation section 3 including a gas phase outlet 13, an effluent flow from the upflow reaction zone, for degassing thereby reducing a turbulence of the degassed effluent flow, and guiding 130 the degassed effluent flow from the first separation zone 31 along a second separation zone 32 of the separation section 3 separating the degassed effluent flow having reduced turbulence in a lower liquid phase 'LP' comprising a first phase liquid (typically comprising biocatalyst and water) and an upper liquid phase 'UP' comprising reaction product as or in a second phase liquid, wherein at least part of the upper liquid phase is discharged via a second separator outlet 15 of the separation section 3 for collection 150 of formed product, and wherein the lower liquid phase potentially portions of the upper liquid phase not discharged is fed to a recycle flow section inlet 16 of recycle flow section 4 of the integrated multiphase biocatalytic reactor and separator system 1 via a first separator outlet 14, and; and recirculating 140 the lower liquid phase 'LP' via the recycle flow section 4 back to the upflow reaction section.

It will be appreciated that in the process as described herein the first phase liquid acts as a preferential carrier for the biocatalyst and the substrate providing a reaction medium for the biocatalytic conversion to desired products and that the second phase liquid comprises or essentially consists of formed product or that the second phase liquid can additionally include a preferential carrier (solvent) for receiving formed product. Such carrier may also be referred to as a product recovery phase or extraction medium.

It will be understood that the method according to the invention can, particularly during a continuous operation mode, further comprise the steps of adding 101 one or more flows to constituents comprised in the reaction mixture, e.g. to fill the system during start 'UP' and/or to replenish converted constituents (such a substrate and second phase liquid) and/or to change a relative concentration thereof during an operation cycle.

In a preferred embodiment, the method further comprises comprising controlling or adjusting a residence time in the system, a liquid level at the separation section 3 or both. Inventors found that residence time and/or liquid level can be conveniently adjusted or controlled by one or more of: controlling a flow rate in the recycle flow section 4, e.g. by introducing, removing or replacing one or more flow restriction elements 90 acting on the recycle flow section or setting a flowrate on an adjustable one; and adjusting a lifting force within the upflow reaction section 2 e.g. by adjusting a gas flow rate.

To affect liquid-liquid phase separation efficiency the method may further comprise inserting, replacing or removing (when present) one or more of the coalescence members 70 within the settling zone upstream the first and second separator outlet as described herein above; and/or inserting, replacing or removing (when present) the flow guiding element 80 at an interface between the upflow reaction section 2 and the separation section 3.

As will be recognized from the specification the present integrated multiphase biocatalytic reactor and separator system advantageously allows control over relevant operating parameters including: a recirculation rate; flow regime (as defined by Reynolds number Re) within the upflow reaction section 2 and separation section 3, particular in the second separation zone 32; a liquid level in the separation section. Conjointly these parameters can control a residence time or separation efficiency within the separation section 3.

Recirculation can advantageously be controlled by aeration rate, optional stirring, and/or chemical composition of the reactor contents (e.g. viscosity (biomass density) and polar effects of salts). Friction along inner walls of the system is generally minimized as much as possible. To increase friction within the system, e.g. to reduce flow, various modular elements as described herein can be introduced into the system as desired (e.g. restriction members, flow guiding elements, and the like). Preferably, a height of the reaction mixture in the upflow reaction section 2 is at least 25 cm to create enough driving force to enable recirculation. Inventors found that for effective liquid-liquid phase segregation a laminar flow profile (Re < 2000) within at least the second separation zone 32 is strongly preferred. Upstream (in the first separation zone 31) a more turbulent flow profile can be accepted. Inventors found the flow profile within the second separation zone 32 correlates with the LP liquid level (H_sep) within the separator, whereby turbulence decreases with increasing height of the liquid level. Inventors determined empirically that an essentially laminar flow profile in combination with recirculation can be obtained when LP liquid level is ≥ about 2 cm. As known in the field, turbulence in the upflow reaction section 2 (fermentation compartment or reaction zone) is primarily set by gas flow requirements as follows from liquid side mass transfer coefficient k_{L}a (which can be a transfer coefficient for example for oxygen in aerobe processes or for example for carbon dioxide CO2 in anaerobe process conditions) and fermentation process requirements.

To improve an overall reaction rate per reactor volume (e.g. fermentation rate) the volume of upflow reaction section 2 (V₂) relative to the volume of the separation section 3 (Vs) is preferably as large as possible, i.e V₂/V₃ is preferable as large as possible. However, for separation and for sampling the separation section volume is preferable above a minimum threshold value. As outlined above this volume can be minimized by addition of the modular elements disclosed herein, such the overflow element (50) which protects the harvesting position against turbulence for instance in the form of a draw off box with a weir. From a practical perspective to assure separation a residence time within the separator is required of at least 1 minute. Accordingly, the ratio V₃/V₂ is preferably within a range of 0.2 - 1.0.

Further, it will be appreciated that biocatalysts, substrates, nutrients, additional liquid phases (for extracting a product from the reaction mixture) operating conditions etc, can be based on the art, such as described in the above cited prior art, in particular WO2021/010822 of which the contents, in particular the claims, are incorporated herein by reference.

The organic substance produced in accordance with the invention can in principle be any kind of organic substance that can be produced using a biocatalyst. The organic substance can be a substance having 1 carbon or more carbons. Usually, the organic substance has at least 3 carbons, in particular at least 4 carbons, more in particular at least 5 carbons. The organic substance can have over a 1000 carbons (such as in case of produced biopolymers. In an embodiment the organic substance has up to 100, up to 50, up to 30 or up to 25 carbons.

Advantageously, the method according to the invention can be used to produce one or more organic substances selected from the group consisting of hydrocarbons, in particular monoterpenes, sesquiterpenes, aromatic hydrocarbons; isoprenoids (terpenoids); organic acids, in particular C5-C24 fatty acids, more in particular C12-C20 fatty acids; alcohols, in particular alcohols having at least 4 carbon atoms; ketones, in particular ketones having at least 5 carbon atoms; aldehydes in particular aldehydes having at least 5 carbon atoms; cyclic carboxylic esters, in particular lactones; non-cyclic esters, in particular non-cyclic esters having at least 5 carbon atoms; lipids in particular glycerides; ethers; pyridines; imines; imides; amines; amino acids; and peptides. For further details of preferred organic substances that can in be produced in accordance with the invention, reference is made to the prior art cited herein, in particular WO2021/010822 page 23, line 27 till page 27, line 10 of which the contents are incorporated herein by reference.

Advantageously, the biocatalyst comprises a micro-organism selected from the group of bacteria, archaea and fungi, preferably selected from the genera *Pseudomonas*, *Gluconobacter*, *Rhodobacter*, *Clostridium*, *Escherichia, Paracoccus, Methanococcus, Methanobacterium, Methanocaldococcus* , *Methanosarcina, Aspergillus, Penicillium, Saccharomyces, Kluyveromyces, Pichia, Candida, Hansenula, Bacillus, Corynebacterium, Blakeslea, Phaffia (Xanthophyllomyces), Yarrowia, Schizosaccharomyces, Zygosaccharomyces, Saccharopolyspora* and *Zymomonas* more preferably from the group of *Corynebacterium glutamicum, Escherichia coli, Bacillus subtilis* , *Bacillus methanolicus, Pseudomonas aeruginosa, Pseudomonas putida, Rhodobacter capsulatus, Rhodobacter sphaeroides, Paracoccus carotinifaciens, Paracoccus zeaxanthinifaciens, Saccharomyces cerevisiae, Saccharomyces pastorianus, Schizosaccharomyces pombe, Aspergillus nidulans, Aspergillus niger, Aspergillus oryzae, Blakeslea trispora, Penicillium chrysogenum, Phaffia rhodozyma (Xanthophyllomyces dendrorhous), Pichia pastoris, Yarrowia lipolytica, Saccharopolyspora spinosa and Zymomonas mobilis,* in particular from the group of *Escherichia coli, Pseudomonas aeruginosa, Pseudomonas putida, Saccharomyces cerevisiae, Saccharopolyspora spinosa and Zymomonas mobilis.* A biocatalyst selected from this group may in particular be used for the production of a substance according to the previous paragraph.

A liquid phase for extracting (recovering) the produced substance of interest forms a phase separate from the biocatalytic liquid phase. Particularly suitable liquids for the product recovery phase are organic liquids, in particular hydrophobic organic liquids. These may in particular comprise one or more compounds selected from the group of hydrocarbons, organic acids, alcohols, ketones, aldehydes, cyclic carboxylic esters, non-cyclic esters, lipids, amines, including mixtures thereof. Preferred are alkanes, triglycerides and hydrophobic alcohols. Particularly preferred alkanes have 6 carbons (C6) or more, more preferably C7-C25, e.g. C7-C15. Specific examples of particularly suitable liquid alkanes are hexanes, heptanes, octanes, nonanes, decanes, dodecanes and pentacosane. The alkane may be linear or branched or cyclic. Good results have, amongst others been achieved with dodecane. Further, liquid hydrophobic alcohols are particularly suitable. Preferably the alcohol is selected from C8 alcohols and higher, more preferably C10-C20 alcohols. The alcohol may be linear or branched or cyclic. Specific examples of particularly suitable alcohols are an octanol, a decanol, a dodecanol, oleyl alcohol and isomeric mixtures thereof. Further, liquid triglycerides are particularly suitable, preferably vegetable oils. Particularly preferred examples are castor oil, sunflower oil and soy(bean) oil. The various examples of liquids for the recovery phase that are given are not or poorly soluble in water. Furthermore they typically show good biocompatibility, which is of interest when using a living cell for the biocatalysis.

If desired, the liquid phase comprising the product of interest can be subjected to further downstream processing. This can be done in a manner known per se, e.g. by back extraction to a lower boiling solvent, and/or solvent evaporation as described in (WO2021/010822).

For the purpose of clarity and a concise description, features are described herein as part of the same or separate embodiments, however, it will be appreciated that the scope of the invention may include embodiments having combinations of all or some of the features described. For example, while embodiments were shown for biocatalytic reactions, also alternative ways such as processes using different catalysts or even non-catalytic processes may be envisaged by those skilled in the art having the benefit of the present disclosure for achieving a similar function and result. The various elements of the embodiments as discussed and shown offer certain advantages, such as efficient recovery of a biocatalytically produced substance, even when the concentration of produced substance in the reaction mixture is kept at a relatively low level in the aqueous phase (such as a fermentation broth), while offering reliable control over process conditions. Whereas the recycle flow section 4 in some embodiments is depicted as an external loop section it will be appreciated that recycle flow section 4 may also be provided as being adjacent or adjoining to an external wall of the upflow reaction section (e.g. as described in relation to FIG 7C) or even by a conduit extending partially within the upflow reaction volume. Routing the recycle flow adjacent, adjoining or even within the upflow reaction section can advantageously reduce thermal losses and/or mitigate temperature variations within the system.

Of course, it is to be appreciated that any one of the above embodiments or processes may be combined with one or more other embodiments or processes to provide even further improvements in finding and matching designs and advantages.

## Claims

1. An integrated multiphase biocatalytic reactor and separator system (1), comprising one or more inlets (11) for feeding reactor contents, an upflow reaction section (2), a separation section (3), and a recycle flow section (4), wherein:
the upflow reaction section (2) comprises a gas phase inlet (12), which gas phase inlet is arranged to introduce a gas phase (G),
the separation section (3) is configured to direct an effluent flow received from the upflow reaction section in a lateral direction with respect to a net flow direction in the upflow reactor section, along a first separation zone (31) and a second separation zone (32) ,
the first separation zone (31) is at least configured for degassing the effluent flow,
the second separation zone (32) which is a liquid-liquid phase separator configured for receiving a degassed effluent flow from the first separation zone (31) and which is at least configured to separate the degassed effluent flow in a lower liquid phase (LP) and an upper liquid phase (UP),
the separation section (3), further comprising a gas phase outlet (13),
the separation section (3) further comprising a first separator outlet (14) for the lower liquid phase and a second separator outlet (15) for discharging the upper liquid phase (UP); and
the recycle flow section (4) comprises a recycle flow section inlet (16) for the lower liquid phase (LP) that is fluidly connected to the first separator outlet (14) for the lower liquid phase and a recycle flow section outlet (17) opening into the upflow reaction section (2).

2. The reactor and separator system according to claim 1, wherein the recycle flow section outlet (17) opens at a bottom section of the upflow reaction section (2) and wherein the recycle flow section inlet (16) is positioned higher than the recycle flow section outlet (17) at a position to, in use, allow recirculating the lower liquid phase (LP) by hydrostatic forces.

3. The reactor and separator system according to claim 1 or 2, wherein the separation section (3) includes a flow guidance panel (40) extending within a downstream portion of the second separation zone 32 to guide the lower liquid phase (LP) flow towards the first separator outlet (14).

4. The reactor and separator system according to any of the preceding claims, comprising an overflow element (50) at the second separation zone (32) upstream of the second separator outlet (15) configured to restrict passage of lower liquid phase at a liquid level (L) exceeding an overflow edge (51).

5. The reactor and separator system according to any of the preceding claims, further comprising a barrier (60) extending upwardly within the separation section (3) from a base (3b) of separation section (3), arranged to reduce turbulence of the effluent flow.

6. The reactor and separator system according to claim 5, wherein the barrier includes a downstream portion (61) that gradually slopes toward the base (3b).

7. The reactor and separator system according to any of the preceding claims 5 or 6, wherein the barrier (60) includes a slit 62 separating a lower section 63 extending to the base (3b) and an upper section 62 extending to an upper bound (3u) of the separation section (3).

8. The reactor and separator system according to any of the preceding claims, comprising a flow guiding element (80) at an interface between the upflow reaction section (2) and the separation section (3), the flow guiding element (80), preferably including a first flow guiding element (81) providing a first volume with a reduced cross-sectional area above the upflow reaction section (2) and a second flow guiding element (82) having a second volume that extends laterally from the first and which has a gradually increasing cross-sectional area to open into the second separation zone (32).

9. The reactor and separator system according to any of the preceding claims, wherein the separation section (3) zone includes a coalescence promoting element (70) extending along a flow path and crossing a phase boundary between the upper and lower liquid phases during operation within the second separation zone (32).

10. The reactor and separator system according to any of the preceding claims, comprising one or more removable or adjustable restriction elements (90) to flow reduce a flowrate of the lower liquid phase 'LP' in the recycle flow section (4).

11. The reactor and separator system according to any of the preceding claims, wherein one or more of the flow guidance panel (40), the barrier (60) and the coalescence promoting element (70) are coated with or formed of a hydrophobic or lyophilic material.

12. The reactor and separator system according to any of the preceding claims, wherein the liquid-liquid phase separator comprises a flow element comprising a downward section (32-1) for conveying the degassed effluent flow towards a diverging connection member (32-2) which is connected the first separator outlet (14) and to an upward section (32-3) for conveying the lower liquid phase (LP) to the second separator outlet (15).

13. The reactor and separator system according to any of the preceding claims, wherein the system is arranged in core-shell configuration comprising an inner tube section (2-1) defining the upflow reaction section (2), surrounded by an outer annulus (4-1) defining the recycle flow section (4).

14. A method for biocatalytic conversion of a substrate to a product comprising:
flowing a reaction mixture comprising at least a liquid carrier, a substrate and a biocatalyst upwards through the upflow reaction section (2) of the reactor and separator system according to any of the claims 1-13 towards the separation section (3)
providing a driving force for lifting the reaction mixture by feeding a gas phase (G) into the upflow reaction section (2);
receiving, at the first separation zone (31) of the separation section (3) including the gas phase outlet (13), an effluent flow from the upflow reaction zone,
degassing the received effluent flow in the first separation zone (31) thereby reducing a turbulence of the degassed effluent flow, and
guiding the degassed effluent flow from the first separation zone (31) along the second separation zone (32) of the separation section (3), separating the degassed effluent flow into a lower liquid phase (LP) and an upper liquid phase (UP), said lower liquid phase enriched in the liquid carrier compared to the degassed effluent, said upper liquid phase enriched in formed reaction product, wherein the lower liquid phase is fed to a recycle flow section inlet (16) of the recycle flow section (4) of the integrated multiphase biocatalytic reactor and separator system (1) via the first separator outlet (14), and wherein at least part of the upper liquid phase is discharged via the second separator outlet (15) of the separation section (3); and
recycling the lower liquid phase (LP) via the recycle flow section (4) back to the upflow reaction section (2).

15. The method according to claim 14, comprising adjusting a residence time in the system, a liquid level at the separation section (3) or both.

16. A use of the reactor and separator system according to any of the claims 1-13 to apply selection pressure for selection of one or more micro-organism strains, comprising applying the method according to claim 14 or 15.
